# EUROPEAN PATENT APPLICATION

(11) **EP 3 919 108 A1**
(43) Date of publication of application: **08.12.2021**
(21) Application number: 21179841.8
(22) Date of filing: 19.05.2020
(51) Int. Cl.: A61M 16/06, A61M 16/08

(54) **NASAL CANNULA AND TUBING**

(30) Priority: 10.06.2019 US 201916436615
(62) Divisional of application: 20175551.9
(71) Applicant: Neotech Products LLC, Valencia, CA 91355 (US)
(72) Inventor: McNally, Matthew, Valencia, 91355 (US); Mitchell, Richard, Valencia, 91355 (US); Kiliszewski, Lawrence, Valencia, 91355 (US); McCrary, Craig, Valencia, 91355 (US)
(74) Representative: RGTH

(57) **Abstract**

A gas supply tube for use in medical settings for the supply of gas to a patient. The airway tube has a generally semi-circular cross-section so as to provide a flat exterior surface for abutment or contact with a patient's skin. A nasal cannula system is constructed using the gas supply tube has a nasal body with cannula configured for insertion into nasal openings of a patient. The nasal body is connected to one or more gas supply or airway tubes. Both the nasal body and the airway tubes have a semi-circular cross-section. The other end of the airway tube(s) is connected to one or more supply adapters having ports matching the semi-circular cross-section. A surface retainer with a clamp having a semi-circular cross-section may adhere the airway tube to a patient's skin.

## Description

### BACKGROUND OF THE INVENTION

The present invention is directed to airway delivery systems used in medical treatment, and more particularly to tubing and connections for the system having a specific shape. The shape of the tubing and connections is intended to provide a more comfortable and stable placement of the airway delivery systems on and around a patient, particularly their face.

Nasal continuous positive airway pressure, NCPAP, is a standard used for administration of non-invasive positive airway pressure, particularly in the Neonate. Administration of non-invasive positive airway pressure is usually accomplished by tubing being run from behind or above a patient's head to their nose where cannula are inserted into the nasal openings. The tubing is generally run above a patient's ears and across the cheeks.

Typical tubing and connections for such systems have a circular cross-section. One drawback of tubing and connections with a circular cross-section is that the same can easily slide and/or roll relative to the face or head of the patient. When the tubing and connections slide and/or roll, such can tend to become dislodged from the nasal openings and cease to provide airflow. Another drawback is that such circular tubing can create pinch and pressure points when a patient may lay on the tubing. Such drawbacks can be exacerbated when the systems are used in neonatal settings. Infant patients cannot be given direction or instruction to not move and avoid dislodging ort pinching the tubing and connections.

Accordingly, there is a need for tubing and connections for use in airway delivery systems that minimize the risk of sliding, moving, dislodging, and pinching through patient movement during use, particularly in neonatal patients. The present invention fulfills these needs and provides other related advantages.

### SUMMARY OF THE INVENTION

The present invention is directed to a nasal cannula system having a nasal body with a pair of nasal prongs configured for insertion into nasal passages when worn by a patient. An elongated gas delivery tube is communicatingly connected to the nasal body, wherein the gas delivery tube has in cross-section a generally flat side joined to a generally curved side that together form a passageway therebetween having a semi-circular cross-section. An internal rib extends through the passageway along a length of the gas delivery tube, spanning from the generally flat side to the generally curved side.

The internal rib may be continuous along the length of the gas delivery tube so as to divide the gas delivery tube into two separate passageways. Alternatively, the internal rib may be discontinuous along the length of the gas delivery tube so as to allow fluid communication between areas of the passageway on opposite sides of the internal rib. The gas delivery tube preferably has a generally flat exterior side configured to lay against a patient's skin when worn.

The system may further include a surface retainer with a clamp, wherein the surface retainer is configured for adherence to a patient's skin. The claim has a semi-circular cross-section configured to slidingly receive and hold the gas delivery tube against the patient's skin when worn.

The system further has a supply adapter having a gas port with a semi-circular cross-section matching that of the passageway. The supply adapter preferably comprises a connection port with a semi-circular cross-section matching that of the gas delivery tube.

In a particularly preferred embodiment, the gas delivery tube is a pair of gas delivery tubes, each of said pair of gas delivery tubes configured to lay against opposite sides of a patient's head when worn. The system further has a supply adapter with a pair of gas ports each having a semi-circular cross-section matching that of the passageway of one of the pair of gas delivery tubes. Alternatively, the supply adapter may have a connection port with a generally circular cross-section configured to receive the pair of gas delivery tubes with flat exterior sides abutting. Still alternatively, the supply adapter may have a pair of connection ports, each of the pair of connection ports having a semi-circular cross-section matching that of one of the pair of gas delivery tubes.

The present invention is also directed to a gas supply tube for medical delivery of gases. The inventive gas supply tube has an elongated body with a generally flat side joined to a generally curved side disposed opposite the flat side. The flat side and the curved side together form a passageway having a generally semi-circular cross-section, wherein an exterior surface of the flat side is generally flat. An internal rib extends along a length of the elongated body through the passageway, spanning from the flat side to the curved side.

The internal rib may be continuous along the length of the elongated body so as to divide the passageway into two separate passageways. Alternatively, the internal rib may be discontinuous along the length of the elongated body so as to allow fluid communication between regions of the passageway on opposite sides of the internal rib.

Other features and advantages of the present invention will become apparent from the following more detailed description, taken in conjunction with the accompanying drawings, which illustrate, by way of example, the principles of the invention.

### BRIEF DESCRIPTION OF THE DRAWINGS

The accompanying drawings illustrate the invention. In such drawings:
FIGURE 1 is an environmental view illustrating an application of a first preferred embodiment of the inventive nasal cannula system as attached to an infant patient;
FIGURE 2 is a perspective view of the first preferred embodiment of the inventive nasal cannula system;
FIGURE 3 is an environmental view illustrating an application of a second preferred embodiment of the inventive nasal cannula system as attached to an infant patient;
FIGURE 4 is a perspective view of the second preferred embodiment of the inventive nasal cannula system;
FIGURE 5 is a close-up perspective view of the connection between a tube and a connector of a preferred embodiment of the inventive nasal cannula system;
FIGURE 6 is a perspective view of a length of a first preferred embodiment of the inventive gas supply tube;
FIGURE 7 is a perspective view of a length of a second preferred embodiment of the inventive gas supply tube;
FIGURE 8A is a perspective view of a length of gas supply tube being inserted into a supply adapter;
FIGURE 8B is a cut-away perspective view of a length of gas supply tube being inserted into a supply adapter;
FIGURE 9A is a perspective view of lengths of a pair of gas supply tubes being inserted into a supply adapter; and
FIGURE 9B is a cut-away perspective view of lengths of a pair of gas supply tubes being inserted into a supply adapter.

### DETAILED DESCRIPTION OF THE PREFERRED EMBODIMENTS

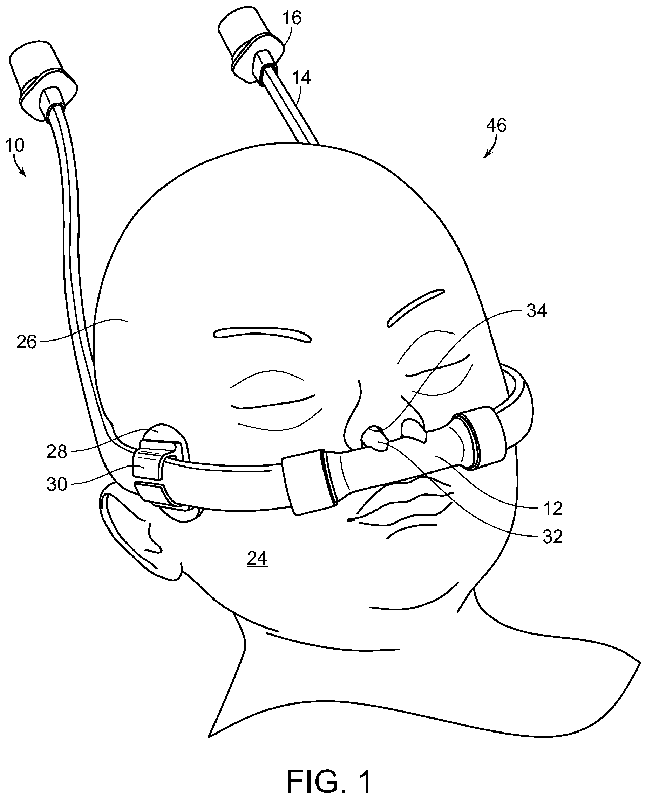

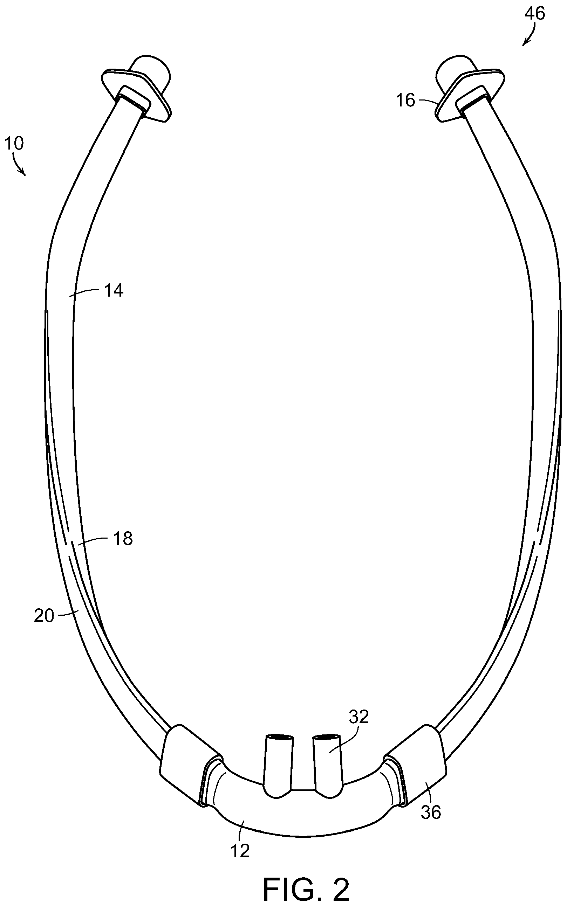

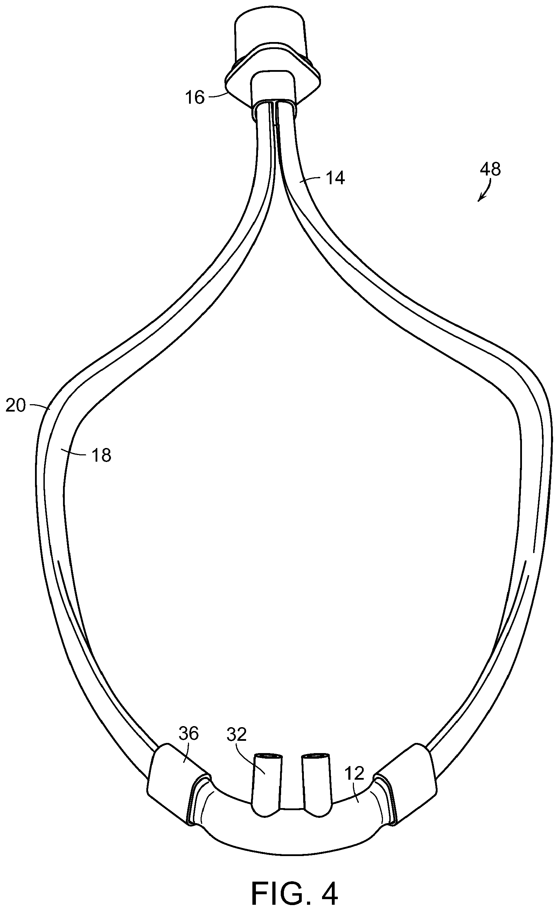

In the following detailed description, the nasal cannula system of the present invention is generally referred to by reference numeral 10 in FIGS. 1-4. The primary components of the system 10 represent the nasal body 12, the airway tubing 14, and the supply adapter 16.

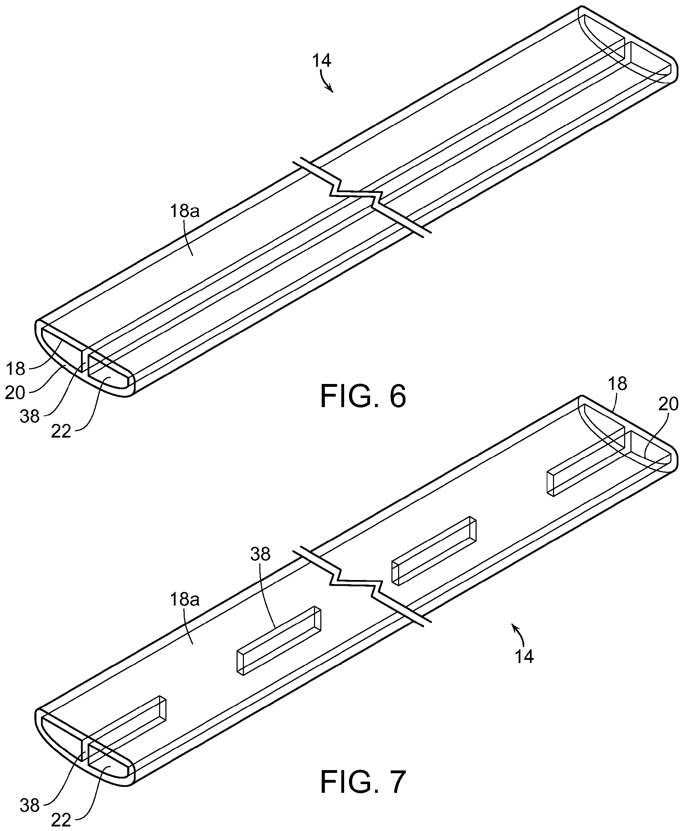

The airway tubing 14, shown in transparent form in FIGS. 6 and 7, has a generally semi-circular cross-section. The tubing 14 has a generally flat side 18 and a generally curved side 20 that a connected to form the semi-circular cross-section. The flat side 18 is flat on at least an exterior surface 18a relative to the tubing 14, but is preferably flat on an interior surface as well. Similarly, the curved side 20 is curved on at least an exterior surface 20a relative to the tubing 14, but is preferably curved on an interior surface as well.

When the flat side 18 and curved side 20 are combined, the exterior surfaces 18a, 20a combined to create a generally semi-circular cross-section on the exterior of the tubing 14. Similarly, when the interior surfaces of the flat side 18 and curved side 20 are flat and curved, respectively, they form a passageway 22 through the tubing 14 that has a semi-circular cross-section.

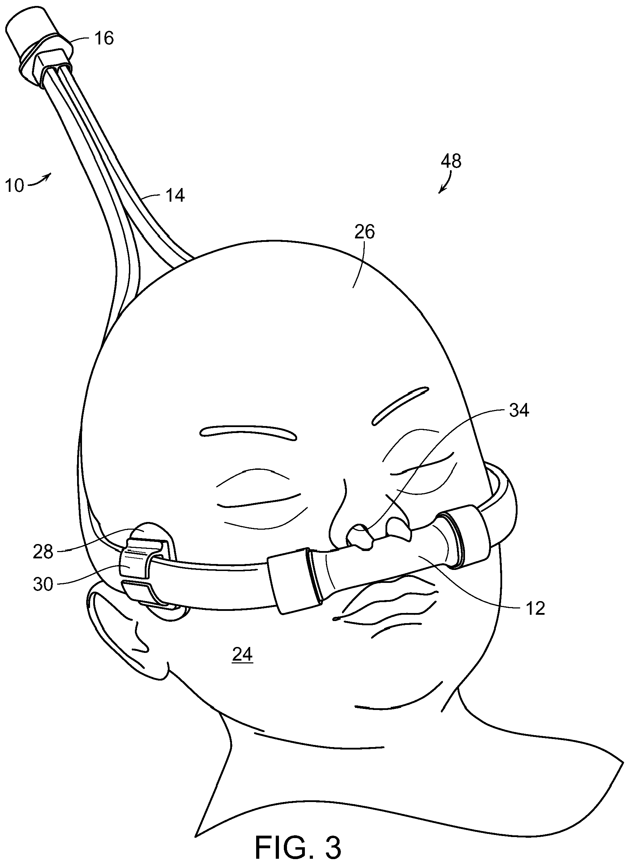

The flat exterior surface 18a of the flat side 18 is configured to lay flush against the skin of a patient when the tubing 14 is used. In this way, the tubing 14 has a lower profile when resting against the skin 24 of a patient 26, e.g., on the cheek or otherwise around the face or head, as shown in FIGS. 1 and 3. This lower profile minimizes the degree to which a patient, particularly an unconscious or an infant patient, may disturb or dislodge the tubing 14 or system 10 due to interference from hand or arm movement near the tubing 14 or contact with other objects, e.g., pillows, due to movement of the head. The lower profile and exposed exterior curved surface 20a also minimizes pinching or pressure points when the tubing 14 may be pressed between the patient's skin and an external object, i.e., a pillow or other medical device.

The flat exterior surface 18a also minimizes the degree to which the inventive tubing 14 may roll, slide, or otherwise be displaced when in use on a patient. The flat exterior surface 18a provides a stable surface with increased contact against the skin 24 of a patient. Such stable surface minimizes rolling and the increased contact minimizes sliding or other movement across the skin 24. To assist in this effort, the system 10 may include a surface retainer 28 that is configured to removably adhere to the skin 24 of a patient 26. The surface retainer has a clamp portion 30 is slightly raised above the skin 24 and has a semi-circular cross-section that generally matches the semi-circular cross-section of the tubing 14.

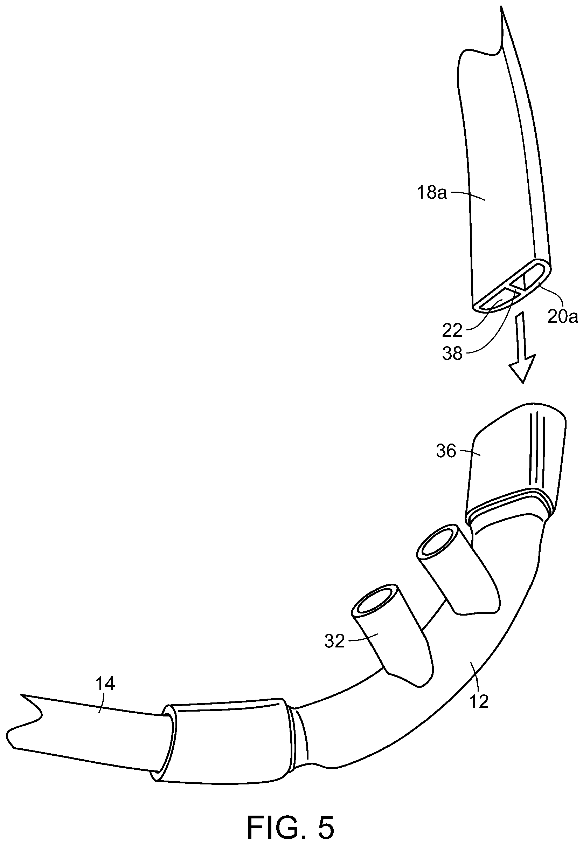

As shown in FIG. 5, the nasal body 12 comprises an elongated tube and preferably has a flat exterior surface 12a that is positioned so as to lay flush against the patient's skin 14, e.g., on the upper lip beneath the nose. The nasal body 12 includes a pair of nasal prongs 32 that are configured to enter nasal openings 34 when in use. The nasal body 12 also includes at least one tube connector 36 at one end of the body 12 for receipt of airway tubing 14. The tube connector 36 also has a generally flat exterior surface 36a that matches the flat exterior surface 12a of the nasal body 12. The tube connector 36 also preferably has a semi-circular cross-section that matches the semi-circular cross-section of the airway tubing 14.

Where the nasal body 12 has a single tube connector 36 at one end, the other end of the nasal body is closed off. In this way, airway tubing 14 can introduce oxygen or another gas into the nasal body 12 for passage through the nasal prongs 32 into the nasal openings 34. Preferably, the nasal body 12 has tube connectors 36 opposite ends thereof, each having a generally semi-circular cross-section configured for sliding reception of inventive airway tubing 14. In this way, oxygen or other gases can be introduced into the nasal body 12 for administration to the patient 26.

The tubing 14 preferably has an internal rib 38 that runs the length of the tubing 14 through the passageway 22. The internal rib 38 is designed to provide additional rigidity to the tubing 14 such that the passageway 22 does not become completely closed off or otherwise blocked when the patient 26 may lay on the tubing 14 or other object exerts pressure on the exterior of the tubing 14. The internal rib 38 may be continuous so as to completely divide the passageway 22 into two separate passageways 22a, 22b through the tubing 14.

Alternatively, the internal rib 38 may be discontinuous so as not to completely divide the passageway 22. Periodic gaps in the internal rib 38 allow for all gases to flow to the other side of the discontinuous internal rib 38 when one side of the passageway 22 may become pinched of blocked. In tubing 14 that has a continuous internal wall 38, pinching or blocking of one side of the passageway 22a or 22b may lead to uneven pressure distribution of the gas causing improper administration of the same.

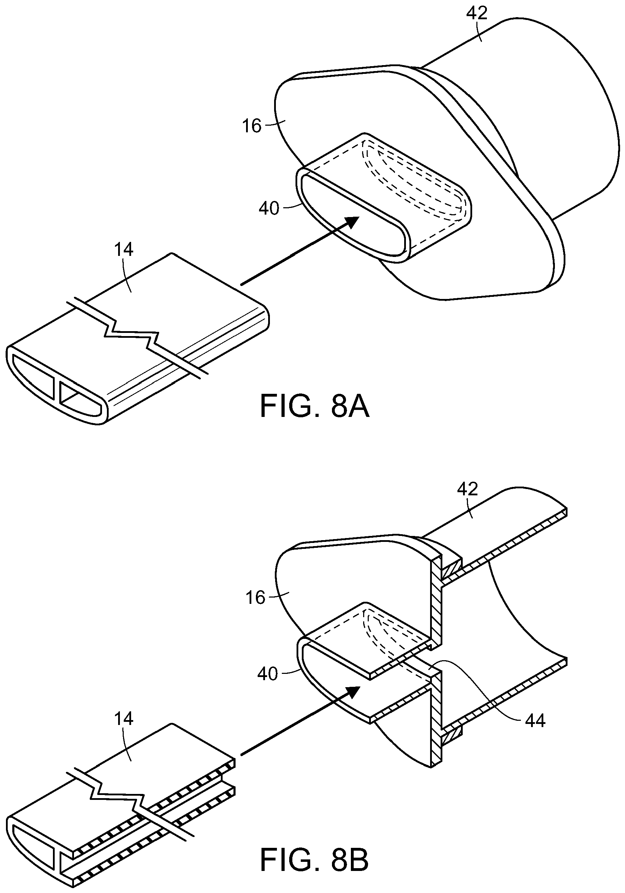

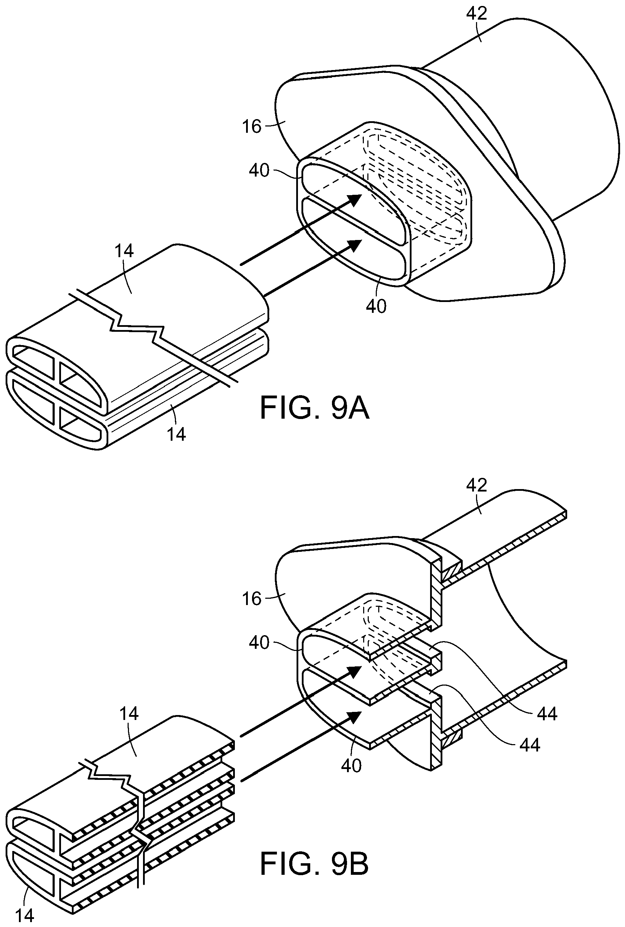

The supply adapter 16, various embodiments shown in detail in FIGS. 8A-9B, generally comprises a connector of coupling with a tube port 40 and a supply port 42 oppositely disposed. The tube port 40 preferably has a semi-circular cross-section that matches the semi-circular cross-section of the airway tubing 14. An opening 44 fluidly connects the tube port 40 to the supply port 42. Preferably, the opening 44 also have a semi-circular shape to match the shape of the tube port 40 and the airway tube 14. The supply port 42 should have a shape to match the connection available on a hospital or medical gas supply (not shown) to which the supply port 42 is configured to connect.

This embodiment of supply adapter 16 may be used in the system 10 in multiple configurations. A single supply adapter 16 can be connected to a single length of airway tubing 14 that is in turn connected to a nasal body that is closed at the opposite end. The single supply adapter 16 is then connected to a gas supply that provides gas for administration to a patient. Alternatively, two supply adapters 16 can be separately connected to different lengths of airway tubing 14, which separate lengths of airway tubing 14 are in turn connected to opposite ends of a nasal body 12. Each separate supply adapter 16 is connected to appropriately positioned gas supply connections to provide gas for administration to a patient. This configuration of two separate supply adapters 16, as shown in FIG. 1, is referred to as an open-ended 46 configuration.

In an alternate embodiment, the tube port 40 on the supply adapter 16 may comprise two tube ports 40 for receiving two separate airway tubes 14 into the same supply adapter 16. In this embodiment, each of the two tube ports 40 may each have separate openings 44 connecting the tube ports 40 to the same supply port 42. The supply port 42 is the same shape as the earlier embodiment and configured for connection to a gas supply. In this embodiment, separate lengths of airway tubing 14 are connected to each of the tube ports 40 on the same supply adapter 16 with opposite ends of the airway tubes 14 connected to the a nasal body 12. The single supply adapter 16 is connected to an appropriately positioned gas supply connection to provide gas for administration to a patient. This configuration of a single supply adapter 16 connected to multiple airway tubes 14, as shown in FIG. 3, is referred to as a close-ended 48 configuration.

The nasal cannula system 10 and gas airway tubing 14 described herein has a number of particular features that should preferably be employed in combination, although the gas airway tubing 14 is useful separately without departure from the scope and spirit of the invention. Although preferred embodiments have been described in detail for purposes of illustration, various modifications may be made without departing from the scope and spirit of the invention. Accordingly, the invention is not to be limited, except as by the appended claims.

## Claims

1. A nasal cannula system, comprising:
a nasal body having a pair of nasal prongs configured for insertion into nasal passages when worn by a patient;
an elongated gas delivery tube communicatingly connected to the nasal body, wherein the gas delivery tube has a generally flat side joined to a generally curved side that together form a passageway therebetween having a semi-circular cross-section; and
an internal rib extending along a length of the gas delivery tube, spanning from the generally flat side to the generally curved side.

2. The nasal cannula system of claim 1, wherein the internal rib is continuous along the length of the gas delivery tube so as to divide the gas delivery tube into two separate passageways.

3. The nasal cannula system of claim 1, wherein the internal rib is discontinuous along the length of the gas delivery tube so as to allow fluid communication between regions of the passageway on opposite sides of the internal rib.

4. The nasal cannula system of claim 1, wherein the gas delivery tube has a generally flat exterior side configured to lay against a patient's skin when worn.

5. The nasal cannula system of claim 1, further comprising a surface retainer with a clamp having a semi-circular cross-section configured to slidingly receive and hold the gas delivery tube against a patient's skin when worn.

6. The nasal cannula system of claim 1, further comprising a supply adapter having a gas port with a semi-circular cross-section matching that of the passageway.

7. The nasal cannula system of claim 6, wherein said supply adapter comprises a connection port with a semi-circular cross-section matching that of the gas delivery tube.

8. The nasal cannula system of claim 1, wherein the gas delivery tube comprises a pair of gas delivery tubes, each of said pair of gas delivery tubes configured to lay against opposite sides of a patient's head when worn.

9. The nasal cannula system of claim 8, further comprising a supply adapter having a pair of gas ports each having a semi-circular cross-section matching that of the passageway of one of the pair of gas delivery tubes.

10. The nasal cannula system of claim 9, wherein said supply adapter comprises a connection port with a generally circular cross-section configured to receive the pair of gas delivery tubes with flat exterior sides abutting.

11. The nasal cannula system of claim 9, wherein said supply adapter comprises a pair of connection ports, each of the pair of connection ports with a semi-circular cross-section matching that of one of the pair of gas delivery tubes.

12. A gas supply tube, comprising:
an elongated body having a generally flat side joined to a generally curved side disposed opposite the flat side, wherein the flat side and the curved side form a passageway with a generally semi-circular cross-section, wherein an exterior surface of the flat side is generally flat; and
an internal rib extending along a length of the elongated body spanning from the flat side to the curved side.

13. The gas supply tube of claim 12, wherein the internal rib is continuous along the length of the elongated body so as to divide the passageway into two separate passageways.

14. The gas supply tube of claim 12, wherein the internal rib is discontinuous along the length of the elongated body so as to allow fluid communication between regions of the passageway on opposite sides of the internal rib.
